9) Europäisches Patentamt
European Patent Office
Office européen des brevets

(11) Publication number: 0 169 009
A2

# EUROPEAN PATENT APPLICATION

2) Application number: 85304728.0

(51) Int. Cl.⁴: C 07 D 211/90, A 61 K 31/44

2) Date of filing: 02.07.85

3) Priority: 17.07.84 GB 8418127
16.08.84 GB 8420840

3) Date of publication of application: 22.01.86 Bulletin 86/4

3) Designated Contracting States: AT BE CH DE FR GB IT LI LU NL SE

(71) Applicant: FISONS plc, Fison House Princes Street, Ipswich Suffolk IP1 1QH (GB)

(72) Inventor: Baxter, Andrew John, 78 Mount Pleasant Keyworth, Nottinghamshire (GB)
Inventor: Dixon, John, Church Farmhouse Main Street Great Dalby, Nr Melton Mowbray Leicestershire (GB)
Inventor: Gould, Kenneth John, 3A Turvey Lane Long Whatton, Leicestershire (GB)

(74) Representative: Craig, Christopher Bradberry et al, Fisons plc 12 Derby Road, Loughborough Leicestershire LE11 0BB (GB)

**4) Novel dihydropyridine derivatives and their production, formulation and use as pharmaceuticals.**

7) There are described compounds of formula I,

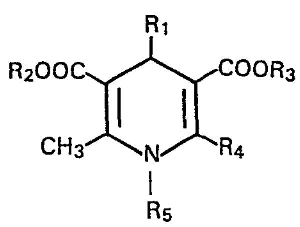

I

in which $R_1$ represents phenyl optionally substituted by ne or more of the groups, halogen, nitro, -CN, or alkyl C1 to optionally substituted by halogen,

$R_2$ and $R_3$, which may be the same or different, each present alkyl C1 to 6 or cycloalkyl C3 to 8,

$R_4$ represents alkyl C1 to 6 substituted by halogen, and $R_5$ represents alkyl C1 to 6.

There are also described methods of making the compounds, and their formulation and use as pharmaceuticals.

# NOVEL DIHYDROPYRIDINE DERIVATIVES AND THEIR PRODUCTION, FORMULATION AND USE AS PHARMACEUTICALS

This invention relates to new compounds, methods for their preparation and compositions containing them.

A wide variety of dihydropyridines have been described as being useful as pharmaceuticals and some, notably nifedipine, have been sold for this use.

We have now found a new group of pyridine derivatives which have pharmacological activity.

According to the invention we provide compounds of formula I,

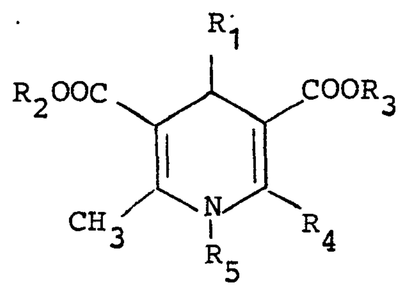

I

in which $R_1$ represents phenyl optionally substituted by one or more of the groups, halogen, nitro, -CN, or alkyl C1 to 6 optionally substituted by halogen,

$R_2$ and $R_3$, which may be the same or different, each represent alkyl C1 to 6 or cycloalkyl C3 to 8,

$R_4$ represents alkyl C1 to 6 substituted by halogen, and

$R_5$ represents alkyl C1 to 6.

According to the invention we also provide the compounds of formula I for use as pharmaceuticals.

According to the invention we further provide a process for the production of a compound of formula I which comprises

a) C1 to 6 alklylation of a corresponding compound of formula I in which $R_5$ is hydrogen, or

b) production of an optical isomer of a compound of formula I by resolution of a mixture of optical isomers of the compound.

The reaction of process a) may be carried out using a suitably alkylating agent, e.g. an alkyl halide, preferably an alkyl iodide, at a temperature of from about $20^{o}$ to $100^{o}C$ in the presence of a suitable solvent, e.g. dimethylformamide or dimethyl sulphoxide. The reaction is preferably carried out in the presence of a base, e.g. potassium hydroxide or sodium hydride.

The resolution of process b) may be carried out by conventional processes known *per se*.

The starting materials for the above processes are either known, or they may be made from known compounds using one or more process steps which are known *per se*.

The compounds of formula I and the intermediates therefor may be isolated from their reaction mixtures using conventional processes, e.g. crystallisation or chromatography.

The compounds of formula I are useful because they

exhibit pharmacological properties in animals. More particularly they block the entry of calcium into vascular and cardiac muscle leading to falls in blood pressure, inotropy and heart rate. They are active in the following systems:-

a) Relaxation of contracted vascular smooth muscle. Van Breeman, Aaronson, Loutzenhiser and Meisheri, Chest, 78, Supplement, 157-165, 1980.

b) Reduction of inotropy and chronotropy of isolated atria. Henry, Excerpta Med. Int. Congr. Ser., 474, 14-23, 1979.

c) Reduction of blood pressure and increase cardiac output in anaesthetised dogs. Hirakawa, Ito, Kondo, Watanbe, Hiei, Banno & Hyase, Arzneim-Forsch, 22, 344-349, 1972.

d) Reduction of blood pressure in conscious dogs when given by the intravenous and oral routes. Newman, Bishop, Peterson, Leroux & Horowitz, J Pharm. Exp. Ther. 201, 723-730, 1977.

The compounds are thus indicated for use in the treatment of renovascular, malignant or essential hypertension (including hypertensive emergencies), pulmonary hypertension, vasospastic angina, chronic stable angina and congestive heart failure. Other indications are the treatment of renal failure, cardiac arrhythmias,

hypertrophic cardiomyopathy, cerebrovascular diseases (including cerebral haemorrhage, ischaemia and vasospasm, migraine, altitude sickness and hearing loss), peripheral vascular diseases (including Raynaud's syndrome, intermittent claudication and digital ulceration); use as a cardioplegic agent during surgery, e.g. in cardiopulmonary bypass, and for the treatment of, and protection against, myocardial infarction and ischaemia.

By virtue of their ability to inhibit calcium entry into other cells and tissues the compounds are also indicated in the treatment of thrombosis, atherosclerosis, respiratory diseases (including asthma and bronchitis) glaucoma, aldosteronism, uterine hypermotility and for the relief of oesophageal and skeletal muscle spasm.

For the above uses the dosage will depend upon the compound used, the route of administration and the effect desired, but in general will be in the range of 0.1-10mg per kilogram body weight per day. For man the indicated total daily dose will be from about 5-500mg, preferably from 5 to 200mg and more preferably from 5 to 100mg, which may be administered preferably once daly, or in divided doses of about 1-200mg, preferably 2 to 25mg, e.g. 2 to 4 times per day.

The compounds of formula I are advantageous in that they possess greater potency (e.g. with respect to

hypotensive and direct negative chronotropic effects), produce a lower level of reflex tachycardia, are more selective (e.g. for vascular smooth muscle vs cardiac muscle), produce less depression of cardiac contractility, are longer acting, are more readily absorbed or less readily metabolised, are more easily formulated, possess less, or less undesirable, side effects, are more stable, (e.g. to light) or have other more beneficial properties than known compounds of similar structure.

The compounds of the invention may be administered by a wide variety of routes and may act systemically or locally. Thus the compounds may be administered by oral or nasal inhalation to the lung, to the buccal cavity, oesophageally, rectally, topically to the skin, the eye or to other available surfaces of the body; by injection, e.g. intravenously, intramuscularly, intraperitoneally, or by surgical implant.

The phenyl in $R_1$ is preferably substituted by one, two or three groups selected from fluoro, chloro, cyano, methyl, and trifluoromethyl.

$R_2$ and $R_3$ are preferably selected from methyl ethyl, isopopyl, cyclopentyl and cyclobutyl. We particularly prefer $R_2$ to be isopropyl, cyclopentyl or cyclobutyl and $R_3$ to be methyl.

We particularly prefer $R_4$ to be mono-fluoromethyl.

$R_5$ is preferably methyl.

According to our invention we also provide a pharmaceutical composition comprising preferably less than 80%, more preferably less than 50%, e.g. 1 to 20%, by weight of a compound of formula I in admixture with a pharmaceutically acceptable adjuvant, diluent or carrier.

Thus the compound may be put up as a tablet, capsule, dragee, suppository, suspension, solution, injection, implant, a topical, e.g. transdermal, preparation such as a gel, cream, ointment, aerosol or a polymer system, or an inhalation form, e.g. an aerosol or a powder formulation.

We prefer compositions which are designed to be taken oesophageally and to release their contents in the gastrointestinal tract. Thus we prefer tablets which may, for example, be made by direct compression. In such a process the active ingredient is mixed with one or more of modified forms of starch, calcium phosphate, a sugar, e.g. lactose, microcrystalline cellulose and/or other directly compressible excipients, together with lubricant(s), e.g. stearic acid or magnesium stearate, flow aid(s), e.g. talc or colloidal silicon dioxide, and disintegrant(s), e.g. starch, substituted sodium carboxymethyl cellulose, cross linked sodium carboxy methyl cellulose, carboxy methyl starch and cross linked polyvinylpyrrolidone. Tablets are then formed by direct

compression, and may be sugar or film coated, e.g. with hydroxpropylmethylcellulose.

Alternatively the active ingredient may be granulated before tabletting. In such cases the active ingredient is mixed with one or more of starch, calcium phosphate, a sugar, e.g. lactose, microcrystalline cellulose or other suitable excipients and granulated with a binder such as starch, pregelled starch, polyvinylpyrrolidone, gelatine, a modified gelatine, or a cellulose derivative, e.g. hydroxypropylmethylcellulose. The mass is then dried, sieved and mixed with lubricant(s), flow aid(s) and disintegrant(s), such as described in the previous paragraph. Tablets are then formed by compression of the granules, and may be sugar or film coated, e.g. with hydroxypropylmethylcellulose.

As a further alternative a powder, blend or granules, such as are described above as intermediates in tabletting, may be filled into a suitable, e.g. gelatine, capsule.

In order to improve the bioavailability, or decrease variability of availability, of the active ingredient the compound may be:-

a) dissolved in a suitable solvent, e.g. polyethylene glycol, Gelucaire, arachis oil, a (hydrogenated) vegetable oil or beeswax and the solution is then filled into a

gelatine capsule,

b) produced as a spray-dried or freeze-dried form prior to mixing with other excipients,

c) milled and/or micronised to produce a powder with a large surface area prior to mixing with other excipients,

d) made into a solution and distributed over an inert excipient having a large surface area, e.g. colloidal silicon dioxide. The solvent is evaporated and further excipients added,

e) formed into a complex with cyclodextrin prior to mixing with other excipients. This complex also assists in increasing light stability, or

f) made into a solid solution or co-precipitated, e.g. with polyvinylpyrrolidone, polyethyleneglycol, modified cellulose, hydroxypropylmethylcellulose, urea or a sugar prior to mixing with further excipients.

The compounds, either in their normal form or in a modified form, e.g. as described immediately above, may be formulated in a controlled release form. Thus the compound may be dispersed, or contained in, a polymer matrix formed from, for example, ethylcellulose, hydroxypropylmethylcellulose or an acrylate/methacrylate polymer. Alternatively the compound may be formulated as a tablet or beads which are surrounded by a semi-permeable membrane, e.g. shellac, ethylcellulose or an acrylate/

methacrylate polymer.

The compounds of this invention may be given in combination with other pharmaceutically active compounds, e.g. diuretics, beta-blockers, antihypertensives or inotropic agents. The dosage of the other pharmaceutically active compound can be that conventionally used when the compound is administered on its own, but is preferably somewhat lower. To illustrate these combinations, a compound of this invention effective in the range, e.g. 5-100 milligrams per day, can be combined at levels ranging. e.g. from 1-200 milligrams per day with the following beta-blockers, antihypertensives nd diuretics in dose ranges per day as indicated:

hydrochlorothiazide (15-200mg), chlorothiazide (125-2000mg), ethacrynic acid (15-100mg), amiloride (5-20mg), furosemide (5-80mg), propanolol (20-480mg), timolol (5-50mg), captopril (10-500mg), methyldopa (65-2000mg) or digoxin (0.1-0.5mg). In addition, the triple drug combinations of hydrochlorothiazide (15-200mg) plus amiloride (5-20mg) plus a compound of this invention (3-200mg) and hydrochlorothiazide (15-200mg) plus timolol (5-200mg) plus a compound of this invention (3-200mg), are provided. The above dose ranges may be adjusted on a unit basis as necessary to permit divided daily dosage. Also, the dose may vary depending on the severity of the

disease, weight of patient and other factors which a person skilled in the art will recognise.

Certain of the compounds of formula I are assymetric and exhibit optical isomers using process b) or may be made by stereospecific syntheses using conventional techniques known per se.

The invention therefore provides the compounds as their individual optical isomers and racemic or other mixtures of the individual isomers.

Certain of the compounds of the invention can form solvates, e.g. hydrates or alcoholates, and certain of the compounds are light sensitive and should therefore be produced, handled, stored and formulated in such a manner that they are not subjected to degrading amounts of light of the appropriate wavelengths.

The invention is illustrated, but in no way limited, by the following Examples, in which temperatures are in °C.

Example 1

3-Methyl 5-(1-methylethyl) 4-[3-chloro-6-fluoro-2-(trifluoromethyl)phenyl]-2-(fluoromethyl)-1,4-dihydro-1,6-dimethyl-3,5-pyridinedicarboxylate

Pulverised potassium hydroxide (0.144g, 2.57mmoles) and iodomethane (80ul, 1.28mmoles) were added with stirring to a solution of 3-methyl 5-(1-methylethyl)

4-[3-chloro-6-fluoro-2-(trifluoromethyl)phenyl]-2-(fluoromethyl)-1,4-dihydro-6-methyl-3,5-pyridinedicarboxylate (0.3g, 0.64mmoles) in dry dimethyl sulphoxide (5ml). After stirring for one hour, the reaction mixture was poured into water and the product was extracted with ethyl acetate (X5). The organic extract was washed with brine, dried ($MgSO_4$) and the solvent evaporated. Chromatography on silica eluting with ether/petroleum ether (60-80$^o$) followed by crystallisation from petroleum ether (60-80$^o$) gave the title compound. mp 94-96$^o$.

The compounds of Examples 2 to 4 were prepared using appropriate starting materials and the method described in Example 1.

Example 2

3-Methyl 5-(1-methylethyl) 4-(2,3-dichlorophenyl)--2-(fluoromethyl)-1,4-dihydro-1,6-dimethyl-3,5-pyridinedicarboxylate

Recrystallised from petroleum ether (60-80$^o$) mp 95-96$^o$.

Example 3

3-Methyl 5-(1-methylethyl) 4-(3-cyanophenyl)-2-(fluoromethyl)-1,4-dihydro-1,6-dimethyl-3,5-pyridinedicarboxylate

Recrystallised from 2-propanol/petroleum ether

(60-80°) mp 91-92°.

Example 4

5-Cyclopentyl 3-methyl 2-(fluoromethyl)-1,4-dihydro--1,6-dimethyl-4-(2-methyl-3-nitrophenyl)-3,5-pyridinedicarboxylate

Recrystallised from 2-propanol/petroleum ether (60-80°) mp 125-6°.

Example A

a) 3-Chloro-6-fluoro-2-(trifluoromethyl)benzaldehyde

Butyl lithium (60.4ml of 1.6M in hexane, 97mmoles) was added with stirring over 1.5 hours under nitrogen to a solution of 1-chloro-4-fluoro-2-(trifluoromethyl)benzene (17.8g, 91mmoles) in dry tetrahydrofuran (150ml) at -73°. After a further 1.5 hours at this temperature, N-methyl-N-phenylformamide (10.86ml, 90mmoles) in dry tetrahydrofuran (20ml) was added over 0.5 hours. After 15 minutes the reaction mixture was poured onto 10% aqueous sulphuric acid. The ethereal layer was separated, washed with saturated sodium bicarbonate, dried ($Na_2SO_4$) and the solvent evaporated. The residue was purified by HPLC eluting with ethyl acetate/petroleum ether 60-80° mixtures. The compound eluted first was discarded.

Further elution afforded 3-chloro-6-fluoro-2-(trifluoromethyl)benzaldehyde (8.35g).

$M^+$ 226/228; nmr ($CDCl_3$) $\delta$ 10.5 (q,H).

b) <u>3-Methyl 5-(1-methylethyl) 4-[3-chloro-6-fluoro-2-(trifluoromethyl)phenyl]-2-(fluoromethyl)-1,4-dihydro-6-methyl-3,5-pyridinedicarboxylate</u>

3-Chloro-6-fluoro-2-(trifluoromethyl)benzaldehyde (1.3g, 5.7mmoles), methyl 4-fluoro-3-oxobutanoate (0.77g, 5.7mmoles) and 1-methylethyl 3-amino-2-butenoate (0.82g, 5.7mmoles) were heated under nitrogen with stirring for 1.5 hours at 90°, followed by 1.5 hours at 100° and then 1 hour at 110°. The cooled reaction mixture was chromatographed twice on silica first using methylene chloride as eluent and then toluene/ethyl acetate mixtures. The sub-title compound (0.2g) was obtained after crystallisation from petroleum ether (60-80°) mp 142-3°.

What we claim is:-

1. A compound of formula I,

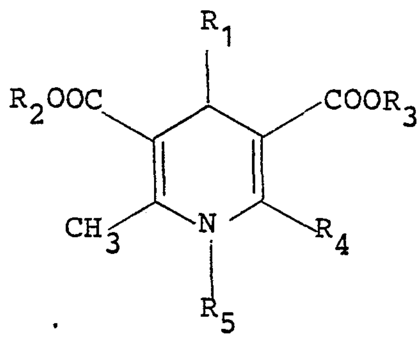

I

in which $R_1$ represents phenyl optionally substituted by one or more of the groups, halogen, nitro, -CN, or alkyl C1 to 6 optionally substituted by halogen,

$R_2$ and $R_3$, which may be the same or different, each represent alkyl C1 to 6 or cycloalkyl C3 to 8,

$R_4$ represents alkyl C1 to 6 substituted by halogen, and

$R_5$ represents alkyl C1 to 6.

2. A compound according to Claim 1, wherein $R_1$ is phenyl substituted by one, two or three groups selected from fluoro, chloro, cyano, methyl, and trifluoromethyl.

3. A compound according to Claim 1 or 2, wherein $R_2$ and $R_3$ are selected from methyl, ethyl, isopopyl, cyclopentyl and cyclobutyl.

4. A compound according to any one of the preceding claims, wherein $R_2$ is isopropyl, cyclopentyl or cyclobutyl, $R_3$ is methyl, and $R_4$ is mono-fluoromethyl.

5. A compound according to any one of the preceding

claims, wherein $R_5$ is methyl.

6. 3-Methyl 5-(1-methylethyl) 4-[3-chloro-6-fluoro-2-(trifluoromethyl)phenyl]-2-(fluoromethyl)-1,4-dihydro-1,6-dimethyl-3,5-pyridinedicarboxylate,

3-Methyl 5-(1-methylethyl) 4-(2,3-dichlorophenyl)--2-(fluoromethyl)-1,4-dihydro-1,6-dimethyl-3,5-pyridinedicarboxylate,

3-Methyl 5-(1-methylethyl) 4-(3-cyanophenyl)-2-(fluoromethyl)-1,4-dihydro-1,6-dimethyl-3,5-pyridinedicarboxylate, or

5-Cyclopentyl 3-methyl 2-(fluoromethyl)-1,4-dihydro--1,6-dimethyl-4-(2-methyl-3-nitrophenyl)-3,5-pyridinedicarboxylate.

7. A pharmaceutical formulation comprising a compound according to any one of the preceding claims in admixture with a pharmaceutically acceptable adjuvant, diluent or carrier.

8. A compound according to any one of Claims 1 to 6 for use as a pharmaceutical.

9. The use of a compound according to any one of Claims 1 to 6 in the production of a pharmaceutical formulation for the treatment of a cardiovascular condition.

10. A process for the production of a compound according to Claim 1, which comprises

a) C1 to 6 alklylation of a corresponding compound of

formula I in which $R_5$ is hydrogen, or

b) production of an optical isomer of a compound of formula I by resolution of a mixture of optical isomers of the compound.

1189J(ir)/jed

What we claim is:-

1. A process for the production of a compound of formula I,

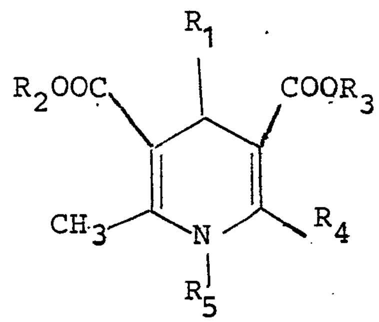

I

in which $R_1$ represents phenyl optionally substituted by one or more of the groups, halogen, nitro, -CN, or alkyl C1 to 6 optionally substituted by halogen,

$R_2$ and $R_3$, which may be the same or different, each represent alkyl C1 to 6 or cycloalkyl C3 to 8,

$R_4$ represents alkyl C1 to 6 substituted by halogen, and

$R_5$ represents alkyl C1 to 6, which comprises

a) C1 to 6 alklylation of a corresponding compound of formula I in which $R_5$ is hydrogen, or

b) production of an optical isomer of a compound of formula I by resolution of a mixture of optical isomers of the compound.

2. A process according to Claim 1, wherein $R_1$ is phenyl substituted by one, two or three groups selected from fluoro, chloro, cyano, methyl, and trifluoromethyl.

3. A process according to Claim 1 or 2, wherein $R_2$ and

$R_3$ are selected from methyl, ethyl, isopropyl cyclopentyl and cyclobutyl.

4. A process according to any one of the preceding claims, wherein $R_2$ is isopropyl, cyclopentyl or cyclobutyl, $R_3$ is methyl, and $R_4$ is mono-fluoromethyl.

5. A process according to any one of the preceding claims, wherein $R_5$ is methyl.

6. A process according to claim 1, wherein the compound of formua 1 is 3-Methyl 5-(1-methylethyl) 4-[3-chloro-6-fluoro-2-(trifluoromethyl)phenyl]-2-(fluoromethyl)-1, 4-dihydro-1,6-dimethyl-3,5-pyridinedicarboxylate.

7. A process according to claim 1, wherein the compound of formua 1 is 3-Methyl 5-(1-methylethyl) 4-(2,3-dichlorophenyl)-2-(fluoromethyl)-1,4-dihydro-1,6-dimethyl-3, 5-pyridinedicarboxylate,

3-Methyl 5-(1-methylethyl) 4-(3-cyanophenyl)-2-(fluoromethyl)-1,4-dihydro-1,6-dimethyl-3,5-pyridinedicarboxylate, or

5-Cyclopentyl 3-methyl 2-(fluoromethyl)-1,4-dihydro-1,6-dimethyl-4-(2-methyl-3-nitrophenyl)-3,5-pyridinedicarboxylate.

8. The use of a compound of formula I as defined in Claim 1 in the production of a pharmaceutical formulation for the treatment of a cardiovascular condition.

1372J(ir)/jed